# EUROPEAN PATENT APPLICATION

(11) **EP 4 056 207 A1**
(43) Date of publication of application: **14.09.2022**
(21) Application number: 20894424.9
(22) Date of filing: 25.11.2020
(51) Int. Cl.: A61L 31/00, A61L 31/04, A61L 31/16, A61M 37/00

(54) **DRUG-LOADED IMPLANTED MEDICAL DEVICE AND PREPARATION METHOD THEREFOR**

(30) Priority: 26.11.2019 CN 201911173102
(71) Applicant: Shanghai MicroPort Medical (Group) Co., Ltd., Shanghai 201203 (CN)
(72) Inventor: LI, Meng, Shanghai 201203 (CN); LI, Junfei, Shanghai 201203 (CN); WANG, Yufu, Shanghai 201203 (CN)
(74) Representative: Arnold & Siedsma
(86) International application number: PCT/CN2020/131420
(87) International publication number: WO 2021/104296

(57) **Abstract**

A drug-loaded implanted medical device (10) and a preparation method therefor. The drug-loaded implanted medical device (10) comprises a device body (100), a hydrophilic coating layer (200) loaded on the device body (100), and crystalline drug particles (300) loaded on the hydrophilic coating layer (200). The hydrophilic coating layer (200) comprises a graft polymer containing a photo-crosslinked group. The medical device (10) uses a hydrophilic coating layer (200) as a carrier, effectively avoiding the risk of embolism, encouraging the crystalline drug particles to fall off, and helping to achieve a target tissue concentration. The invention can also effectively increase the anchoring effect between the carrier and the device, and reduce toxicity.

## Description

### CROSS REFERENCE TO RELATED APPLICATION

The present application claims priority to Chinese Patent Application No. 2019111731022, entitled "DRUG-LOADED IMPLANTED MEDICAL DEVICE AND PREPARATION METHOD THEREFOR", filed on November 26, 2019, the content of which is incorporated herein by reference in its entirety.

### TECHNICAL FIELD

The present disclosure relates to the field of medical materials, and more particularly, to a drug-loaded implantable medical device and a method for preparing the same.

### BACKGROUND

Currently, drug-loaded implantable medical devices have gradually become one of main means for treatment. Especially in cardiovascular diseases, percutaneous coronary intervention (PCI) is currently the main treatment regimen. A drug eluting stent (DES) is still the first choice for PCI. An implantation of DES may prevent blood vessels from rebounding, and an anti-hyperplasia drug loaded on the DES can inhibit a hyperplasia of vascular smooth muscle cells, prevent an intimal hyperplasia, and effectively reduce an incidence of in-stent restenosis. Since the "Sequent Please" of B. Braun Melsungen AG, Germany, was launched in 2004, the drug coated balloon (DCB), as a new interventional treatment technology, has been confirmed by a number of clinical trials for its efficacy and safety in various coronary artery stenosis, small vessel disease, bifurcation disease, etc. The surface of the drug-coated balloon is uniformly coated with anti-hyperplasia drugs. After the drug-coated balloon is delivered to a lesion site, the drug-coated balloon releases the drug within a short expansion time (in a range from 30s to 60s) to inhibit the hyperplasia of vascular smooth muscle cells. Due to the advantages of the drug-coated balloon, such as no implantation during intervention, no risk of thrombosis, and rapid treatment effect, the drug-coated balloon is getting more and more attention.

Currently, an amorphous drug coating is commonly used in the drug-loaded implantable medical device. However, a retention time of the drug released by the drug-loaded implantable medical device in the tissue is very short, so that it is difficult to effectively inhibit the hyperplasia of smooth muscle cells. Although an crystalline drug coating is capable of delaying the release and has an ideal retention time in the tissue, the existing crystalline drug coating has disadvantages that a crystal size is large, the drug is easily broken and dropped when being folded and pressed, and the crystalline drug coating is not easy to fall off the surface of balloon.

### SUMMARY

According to various embodiments of the present disclosure, a drug-loaded implantable medical device and a method for preparing the same are provided.

A drug-loaded implantable medical device includes a device body, a hydrophilic coating carried on the device body, and crystalline drug particles carried on the hydrophilic coating.

In an embodiment, the hydrophilic coating includes a graft polymer containing photo-cross-linking groups.

In an embodiment, the graft polymer containing photo-cross-linking groups is prepared by a graft reaction of a photo-cross-linking monomer and a first polymer.

The photo-cross-linking monomer is at least one of an acrylate-based photo-cross-linking monomer and an acrylamide-based photo-cross-linking monomer.

The first polymer is a hydrophilic polymer with a side chain containing at least one repeating functional group of hydroxyl group, carboxyl group, and amino group.

In an embodiment, the first polymer is selected from at least one of chitosan, dextran, hyaluronic acid, sodium alginate, polyacrylic acid, carbomer, hydroxypropyl methylcellulose, carboxymethylcellulose, polyhydroxyethyl methacrylate, poly (N- (2-hydroxypropyl) methacrylamide), polyarginine, polylysine, and polyaspartate.

In an embodiment, the photo-cross-linking monomer is selected from at least one of acrylate, acrylamide and methacrylate.

In an embodiment, the hydrophilic coating includes a block copolymer formed by a graft polymer containing photo-cross-linking groups and a second polymer. The second polymer is selected from at least one of polyethylene glycol, polyvinyl alcohol, polyvinylpyrrolidone, polyether, polyester, polyamide, polypeptide, and polysaccharide.

In an embodiment, a molar ratio of the graft polymer containing photo-cross-linking groups to the second polymer may be 1: (0.2~1).

In an embodiment, the hydrophilic coating includes a block copolymer formed by a graft polymer containing photo-cross-linking groups, a second polymer, and a third polymer. The second polymer is selected from at least one of polyethylene glycol, polyvinyl alcohol, polyvinylpyrrolidone, polyether, polyester, polyamide, polypeptide, and polysaccharide, and the third polymer is selected from at least one of polyethylene glycol, polyvinyl alcohol, polyvinylpyrrolidone, polyether, polyester, polyamide, polypeptide, and polysaccharide. A molar ratio of the graft polymer containing photo-cross-linking groups, the second polymer, and the third polymer is (0.2~1) :1: (0.2~1).

In an embodiment, a graft ratio of graft polymer is in a range from 20% to 60%.

In an embodiment, the crystalline drug particle has a particle size in a range from 0.5 µm to 5 µm.

In an embodiment, in the crystalline drug particle, a mass percentage of crystalline particles is in a range from 70% to 100%.

A method for preparing a drug-loaded implantable medical device includes:
providing a device body;
preparing a hydrophilic coating solution;
coating the hydrophilic coating solution on the device body to perform a cross-linking reaction, so as to obtain a carrier medical device, and
carrying a drug onto the carrier medical device to obtain a drug-loaded implantable medical device.

In an embodiment, the hydrophilic coating solution includes a graft polymer containing photo-cross-linking groups and a photoinitiator.

In an embodiment, the graft polymer containing photo-cross-linking groups is prepared by a graft reaction of a photo-cross-linking monomer and a first polymer.

The photo-cross-linking monomer is at least one of an acrylate-based photo-cross-linking monomer and an acrylamide-based photo-cross-linking monomer.

The first polymer is a hydrophilic polymer with a side chain containing at least one repeating functional group of hydroxyl group, carboxyl group, and amino groups.

In an embodiment, the first polymer is selected from at least one of chitosan, dextran, hyaluronic acid, sodium alginate, polyacrylic acid, carbomer, hydroxypropyl methylcellulose, carboxymethylcellulose, polyhydroxyethyl methacrylate, poly (N- (2-hydroxypropyl) methacrylamide), polyarginine, polylysine, and polyaspartate.

The photo-cross-linking monomer is selected from at least one of acrylate, acrylamide and methacrylate.

In an embodiment, the hydrophilic coating solution further includes a second polymer. The second polymer is selected from at least one of polyethylene glycol, polyvinyl alcohol, polyvinylpyrrolidone, polyether, polyester, polyamide, polypeptide, and polysaccharide.

In an embodiment, a molar ratio of the graft polymer containing photo-cross-linking groups to the second polymer may be 1: (0.2~1).

In an embodiment, the hydrophilic coating solution further includes a photo-cross-linking monomer. The photo-cross-linking monomer is selected from at least one of acrylate, acrylamide and methacrylate.

In an embodiment, the drug is carried onto the carrier medical device by an immersion method.

In an embodiment, the carrying a drug onto the carrier medical device by an immersion method includes:
preparing a drug solution;
immersing the carrier medical device in the drug solution; and
taking out the immersed carrier medical device, and then immersing the immersed carrier medical device in a crystallization solution for crystallization, and then drying the carrier medical device.

In the above-mentioned drug-loaded implantable medical device, the hydrophilic coating is used as a carrier. Due to the existence of the hydrophilic groups in the hydrophilic coating, more seed crystals may be formed, which is beneficial to form crystalline drug particles with small size, thereby effectively avoiding a risk of embolization. In addition, the introduction of hydrophilic groups may promote an interaction between the carrier and the blood in human body, which is beneficial to promote the falling of crystalline drug particles, so as to help achieve a target tissue concentration and avoid a poor efficacy caused by an extremely low doses of drug. Moreover, the introduction of hydrophilic groups may also effectively increase an anchoring effect between the carrier and the device body, which may prevent the carrier from falling off and reduce toxic and side effects.

### BRIEF DESCRIPTION OF THE DRAWINGS

To illustrate the solutions of the embodiments of the present disclosure or the prior art more clearly, the accompany drawings for describing the embodiments or the prior art are introduced briefly in the following. Apparently, the accompanying drawings in the following description are only some embodiments of the present disclosure, and persons of ordinary skill in the art can derive accompany drawings of the other embodiments from these accompanying drawings without any creative efforts.
FIG. 1 shows a schematic view of a drug-loaded implantable medical device according to an embodiment of the present disclosure.
FIG. 2 is a flowchart of a method for preparing a drug-loaded implantable medical device according to an embodiment of the present disclosure.
FIG. 3 shows a crystal morphology of a drug of a crystalline drug coated balloon containing a drug-loaded implantable carrier of Example 1.
FIG. 4 shows a crystal morphology of a drug of a crystalline drug coated balloon of Comparative Example 1.

### DETAILED DESCRIPTION OF THE EMBODIMENTS

In order to facilitate the understanding of the present disclosure, the present disclosure will be described more fully hereinafter, and preferred embodiments of the present disclosure are given below. However, the present disclosure may be embodied in many different forms and is not limited to the embodiments described herein. Rather, these embodiments are provided, so that the content of the present disclosure could be understood more thoroughly.

All technical and scientific terms used herein have the same meaning as commonly understood by one of ordinary skill in the art to which the present disclosure applies, unless otherwise defined. The terms used in the specification of present disclosure herein are for the purpose of describing specific embodiments only and are not intended to limit the present disclosure. The term "and/or" used herein includes any and all combinations of one or more of the associated listed items.

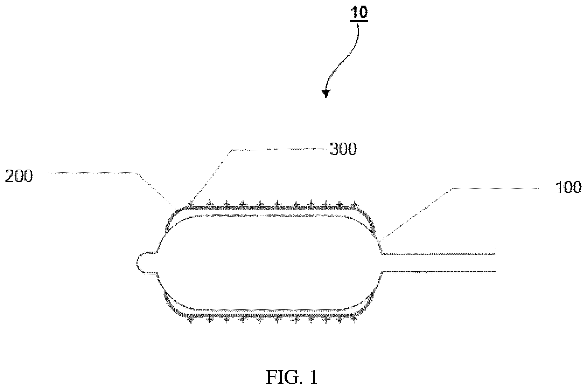

Referring to FIG. 1, according to an embodiment, a drug-loaded implantable medical device 10 is provided, which includes a device body 100, a hydrophilic coating 200 carried on the device body 100, and crystalline drug particles 300 carried on the hydrophilic coating 200.

It could be appreciated that the above drug-loaded implantable medical device 10 may be used in vivo or in vitro, for short-term use or for long-term permanent implantation. Further, the above medical device may be a device that provides the medical treatment and/or diagnosis for cardiac rhythm disorders, heart failure, valvular diseases, vascular diseases, diabetes mellitus, neurological diseases and disorders, plastic surgery, neurosurgery, oncology, ophthalmology, and ENT surgery. The medical devices involved in the present disclosure includes, but are not limited to, stents, stent-grafts, anastomotic connectors, synthetic patches, leads, electrodes, needles, wires, catheters, sensors, surgical devices, angioplasty balloons, wound drainage tubes, shunts, tubes, infusion sleeves, urethral catheters, small balloons, implants, blood oxygenation generators, pumps, vascular grafts, vascular access port, heart valves, annuloplasty rings, sutures, surgical clips, surgical nails, pacemakers, implantable defibrillators, neurostimulators, plastic surgical devices, cerebrospinal fluid shunts, implantable drug pumps, intervertebral cages, artificial discs, replaceable devices for the nucleus pulposus, ear tubes, intraocular lenses and any tubes used in interventional procedures. The stents include, but are not limited to, coronary vascular stents, peripheral vascular stents, intracranial vascular stents, urethral stents, and esophageal stents.

In an embodiment shown in FIG. 1, the above drug-loaded implantable medical device 10 is a drug-coated balloon, that is, the device body is a balloon.

The drug may be selected according to actual needs. For example, the drug may be an anti-proliferative, anti-inflammatory, antiphlogistic, anti-hyperplasia, anti-bacterial, anti-tumor, anti-mitotic, cell-suppressing, cytotoxic, anti-osteoporotic, anti-angiogenic, anti-restenosis, anti-microtubule, anti-metastatic or anti-thrombotic drug. The Drugs include, but are not limited to, dexamethasone, prednisolone, corticosterone, budesonide, estrogen, sulfasalazine and aminosalicylic acid, acemetacin, aescinate, aminopterin, antimycoin, arsenic trioxide, aristolochic acid, aspirin, berberine , bilobol, rapamycin and derivatives thereof (including zotarolimus, everolimus, biomos, 7-O-desmethyl rapamycin, temsirolimus, ridaforolimus, etc.), endothelial statin, angiostatin, angipoietin, monoclonal antibodies capable of blocking the proliferation of smooth muscle cells, levofloxacin, paclitaxel, docetaxel, hydroxycamptothecine, vinblastine, vincristine, doxorubicin, 5-fluorouracil, cisplatin, thymidine kinase inhibitor antibiotics (especially actinomycin-D), hormones, antibody cancer drugs, bisphosphonates, selective estrogen receptor modulators, strontium ranelate, cyclosporine A, cyclosporine C, and brefeldin A.

In an embodiment, a mass percentage of crystalline particles may be in a range from 0% to 100%. Further, the mass percentage of crystalline particles may be in a range from 30% to 100%. Further, the mass percentage of crystalline particles may be in a range from 70% to 100%. The crystalline drug particle may have a particle size in a range from 0.5 µm to 5 µm. Further, the crystalline drug particle may have the particle size in a range from 0.5 µm to 2 µm. In an embodiment, on the drug-loaded implantable medical device 10, a dose of drug may be in a range from 0.8 µg/mm² to 1.2 µg/mm². Further, the dose of drug may be in a range from 0.8 µg/mm² to 1.0µg/mm².

It will be appreciated that the hydrophilic coating 200 contains hydrophilic groups. The presence of hydrophilic groups contributes to a formation of more seed crystals, and thus to a formation of crystalline drug particles with small size, thereby effectively reducing a risk of embolization. In addition, the introduction of hydrophilic groups may promote an interaction between the carrier and the blood in human body, which is beneficial to promote the falling of crystalline drug particles, so as to help achieve a target tissue concentration and avoid a poor efficacy caused by an extremely low doses of drug. Moreover, the introduction of hydrophilic groups may also effectively increase an anchoring effect between the carrier and the device body, which may prevent the carrier from falling off and reduce toxic and side effects.

In an embodiment, the hydrophilic coating 200 includes a graft polymer containing photo-cross-linking groups. The graft polymer containing photo-cross-linking groups is prepared by a graft reaction of a photo-cross-linking monomer and a first polymer.

In an embodiment, the photo-cross-linking monomer is at least one of an acrylate-based photo-cross-linking monomer and an acrylamide-based photo-cross-linking monomer. Further, the photo-cross-linking monomer is selected from at least one of acrylate, acrylamide and methacrylate. In an embodiment, the first polymer is a hydrophilic polymer with a side chain containing at least one repeating functional group of hydroxyl group, carboxyl group or amino group. In an embodiment, the first polymer is selected from at least one of chitosan, dextran, hyaluronic acid, sodium alginate, polyacrylic acid, carbomer, hydroxypropyl methylcellulose, carboxymethyl cellulose, polyhydroxyethyl methacrylate, poly (N- (2-hydroxypropyl) methacrylamide), polyarginine, polylysine, and polyaspartate.

The photo-cross-linking groups are used for grafting to increase the number of reactable sites of the side chains of the polymer, which facilitates an introduction of more hydrophilic groups, and thus facilitates a formation of a network-like structure after a photo-cross-linking reaction, so that it not only improves mechanical properties of the carrier, but also facilitates a formation of more seed crystals when the drug is carried, thereby facilitating a formation of crystalline drug particles with small size and effectively avoiding the risk of embolization. Moreover, an introduction of a large number of hydrophilic groups is beneficial to promote the falling of crystalline drug particles, so as to help achieve a target tissue concentration. Moreover, the introduction of a large number of hydrophilic groups may also effectively increase an anchoring effect between the carrier and the device body, which prevents the carrier from falling off and reduces toxic and side effects.

In an embodiment, a graft ratio of graft polymer is in a range from 1% to 100%. Further, the graft ratio of graft polymer may be in a range from 20% to 60%, or, from 30% to 60%, or, from 20% to 50%, or, from 30% to 50%.

In an embodiment, a molecular weight of the graft polymer containing photo-cross-linking groups is in a range from 1000 to 20000 Daltons.

In an embodiment, the graft polymer is a comb-shaped polymer. The comb-shaped polymer shall be understood to have the common meaning in the art, which refers to a polymer with a comb-like structure, which is formed by regularly and equidistantly grafting a side chain with the same chain length on the same side of a macromolecule main chain. The use of comb-shaped polymer is beneficial to form a polymer with more branches, and these branches may form more interpenetrating cross-linked networks with each other, so that an interaction between the carrier and the device body may be improved, thereby preventing the carrier from falling off and avoiding toxic and side reactions.

In an embodiment, the hydrophilic coating 200 includes a homopolymer and/or block polymer of the graft polymer containing photo-cross-linking groups. It could be appreciated that the homopolymer and/or block polymer containing photo-cross-linking groups refers to that repeating units of the homopolymer and/or block polymer contain photo-cross-linking groups.

In an embodiment, the hydrophilic coating 200 includes a block copolymer formed by the graft polymer containing photo-cross-linking groups and a second polymer. For example, the hydrophilic coating 200 may include an A-B-type block polymer or an A-B-A-type block copolymer formed by the graft polymer B containing photo-cross-linking groups and a second polymer A.

The second polymer may be a hydrophilic polymer containing hydrophilic groups. The hydrophilic polymer containing hydrophilic groups refers to that repeating units of the polymer contains hydrophilic groups such as a hydroxyl group, a carboxyl group, or an amino group. In an embodiment, the second polymer is selected from at least one of polyethylene glycol, polyvinyl alcohol, polyvinylpyrrolidone, polyether, polyester, polyamide, polypeptide, and polysaccharide.

Further, a molecular weight of the second polymer is in a range from 1000 to 10000 Daltons.

In an embodiment, a molar ratio of the graft polymer containing photo-cross-linking groups to the second polymer may be 1: (0.2~1). Further, the molar ratio of the graft polymer containing photo-cross-linking groups to the second polymer may be 1: (0.2-0.5).

In an embodiment, the hydrophilic coating 200 includes a block copolymer formed by the graft polymer containing photo-cross-linking groups, a second polymer, and a third polymer. The third polymer is selected from at least one of polyethylene glycol, polyvinyl alcohol, polyvinylpyrrolidone, polyether, polyester, polyamide, polypeptide, and polysaccharide. For example, the hydrophilic coating 200 may include an A-B-C-type block copolymer formed by the graft polymer B containing photo-cross-linking groups, a second polymer A, and a third polymer C. A molar ratio of the second polymer A, the graft polymer B containing photo-cross-linking groups, and the third polymer C may be (0.2~1) :1: (0.2~1), furthermore, may be (0.2~0.5) :1: (0.2~0.5).

In an embodiment, the drug-loaded implantable carrier 200 is primarily prepared by an ultraviolet cross-linking reaction of the graft polymer containing photo-cross-linking groups, the second polymer and a photo-cross-linking monomer. That is, the photo-cross-linking monomer may also be added during the ultraviolet cross-linking reaction. The photo-cross-linking monomer added at this time may be the same as or different from the photo-cross-linking monomer used in preparing the graft polymer containing the photo-cross-linking groups.

Types of raw materials of the graft polymer containing photo-cross-linking groups and the second polymer, an order of material addition, conditions of ultraviolet cross-linking reaction, and the like, may be adjusted to form various types of network-like hydrophilic coatings, so as to adjust performances of the drug-loaded implantable medical device.

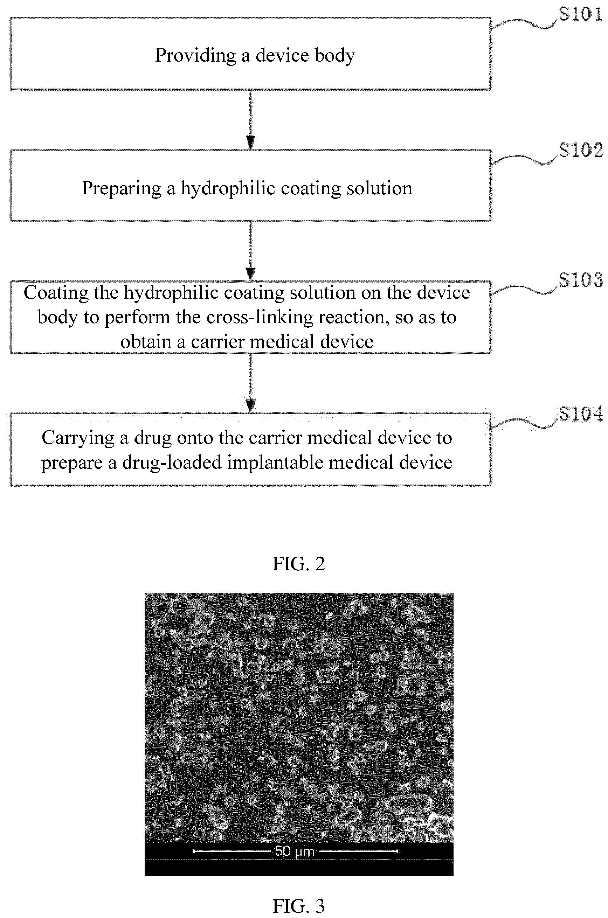

Referring to FIG. 2, a method for preparing a drug-loaded implantable medical device according to an embodiment includes the following steps.

At step S101, a device body is provided.

The device body is as described above, and which is not repeatedly described here.

At step S102, a hydrophilic coating solution is prepared.

In an embodiment, the hydrophilic coating solution includes the above graft polymer containing photo-cross-linking groups and a photoinitiator. In an embodiment, the photoinitiator is a free radical type photoinitiator. The hydrophilic coating solution may be prepared by dissolving the graft polymer containing photo-cross-linking groups and the photoinitiator in a solvent. In an embodiment, the solvent used may be an alcoholic solvent.

In an embodiment, the hydrophilic coating solution further includes a second polymer. The second polymer is selected from at least one of polyethylene glycol, polyvinyl alcohol, polyvinylpyrrolidone, polyether, polyester, polyamide, polypeptide, and polysaccharide.

In an embodiment, the hydrophilic coating solution further includes a photo-cross-linking monomer. The photo-cross-linking monomer is selected from at least one of acrylate, acrylamide and methacrylate. The photo-cross-linking monomer is beneficial to form a network structure, which further improves mechanical properties of the carrier and the interaction of the carrier and the device body, thereby preventing the carrier from falling off.

The graft polymer containing photo-cross-linking groups and the second polymer are as described above and do not be repeatedly described here. The graft polymer containing photo-cross-linking groups may be prepared by a commercially available raw material and by a conventional method, which is not particularly limited herein. In an embodiment, the graft polymer containing photo-cross-linking groups is prepared by a graft reaction of a photo-cross-linking monomer and a first polymer. The first polymer is a polymer with side chains containing groups that can react with the photo-cross-linking monomer. In an embodiment, the first polymer is a hydrophilic polymer with side chains containing at least one repeating functional group of hydroxyl group, carboxyl group and amino group. Further, the first polymer may be selected from at least one of chitosan, dextran, hyaluronic acid, sodium alginate, polyacrylic acid, carbomer, hydroxypropyl methylcellulose, carboxymethylcellulose, polyhydroxyethyl methacrylate, poly (N- (2-hydroxypropyl) methacrylamide), polyarginine, polylysine, and polyaspartate. The photo-cross-linking monomer is at least one of an acrylate-based photo-cross-linking monomer and an acrylamide-based photo-cross-linking monomer. Further, the photo-cross-linking monomer is selected from at least one of acrylate, acrylamide and methacrylate.

In an embodiment, in step S102, a molar ratio of the graft polymer containing photo-cross-linking groups to the second polymer may be 1: (0.2~1). Further, the molar ratio of the graft polymer containing photo-cross-linking groups to the second polymer may be 1: (0.2-0.5). It could be appreciated that a third polymer may also be added during the reaction with the graft polymer containing photo-cross-linking groups. The third polymer is selected from at least one of polyethylene glycol, polyvinyl alcohol, polyvinylpyrrolidone, polyether, polyester, polyamide, polypeptide, and polysaccharide. For example, when the graft polymer B containing photo-cross-linking groups, the second polymer A and the third polymer C are added for reaction, a molar ratio of the second polymer A, the graft polymer B containing photo-cross-linking groups, and the third polymer C may be (0.2~1) :1: (0.2~1), furthermore, may be (0.2-0.5) :1: (0.2-0.5).

At step S103, the hydrophilic coating solution is coated on the device body to perform the cross-linking reaction, and a carrier medical device is obtained after the reaction is completed.

At step S103, the performances of the carrier can be controlled by controlling the time and intensity of the cross-linking reaction. In an embodiment, the cross-linking reaction performed is an ultraviolet cross-linking reaction, and a reaction time is in a range from 30s to 10min. Further, the reaction time may be in a range from 30s to 120s. An intensity of ultraviolet is in a range from 25% to 100%. Further, the intensity of ultraviolet may be in a range from 75% to 100%, which is beneficial to form a better network-like hydrophilic coating.

At step S104, a drug is carried onto the carrier medical device to prepare a drug-loaded implantable medical device.

In step S104, the drug may be carried by an immersion method. For example, step S104 may include: preparing a drug solution; immersing the carrier medical device in the drug solution; taking out the immersed carrier medical device, and then immersing the immersed carrier medical device in a crystallization solution for crystallization, and then drying the carrier medical device.

For the crystallization solution, a suitable solvent combination may be selected according to the type of drug and specific needs, which is not particularly limited here. For example, when the drug is rapamycin, n-hexane may be used to dissolve the drug to prepare a rapamycin solution, and the crystallization may be performed with a mixed solution of ethyl acetate/n-hexane.

The present disclosure will be described in detail hereinafter in combination with the specific examples.

### Example 1

(1) A device body was provided, and the device body of this example was a conventional balloon.
(2) A coating solution was prepared with specific formulations shown in Table 1.
(3) The conventional balloon of the step (1) was immersed in the coating solution of the step (2); the conventional balloon was taken out and placed in an ultraviolet cross-linking apparatus; an ultraviolet cross-linking was performed for 30 s, with an intensity of 100%; and after the ultraviolet cross-linking was completed, the conventional balloon was taken out and dried overnight, thereby obtaining a carrier balloon.
(4) Rapamycin was dispersed in n-hexane and a ultrasonic dispersion was performed for 10 min to prepare a rapamycin solution; the carrier balloon obtained in the step (3) was immersed in the rapamycin solution, and the ultrasonic dispersion was performed for another 10 min; and the balloon was taken out and dried for 2 h, thereby obtaining a drug-loaded implantable balloon.
(5) The drug-loaded implantable balloon of the step (4) was immersed in a clear mixture of rapamycin with ethyl acetate/n-hexane (3:65, v/v) for 5 min; the drug-loaded implantable balloon was taken out and dried naturally for 24 h, and then was sterilized with ethylene oxide to obtain a crystalline drug coated balloon containing a permanent hydrophilic bottom layer (drug-loaded implantable carrier) of Example 1; and a crystal morphology was observed using a scanning electron microscope (SEM), as shown in FIG. 3.

**Table 1**

| Component | | Material Name | Content % (w/w) |
|---|---|---|---|
| Macromolecula r A-B-type block polymer of polymerizable | A | Polyethylene glycol, molecular weight of 1000 | 25% |
| | B | Chitosan, molecular weight of 5000; graft acrylate, grafting rate of 30% | |
| functional groups | | | |
| Photoinitiator | | 2-Hydroxy-4'-(2-hydroxyethoxy)- 2-methy lpheny lacetone | 0.5% |
| Solvent | | Ethanol | 74.5% |

### Example 2

(1) A device body was provided, and the device body of this example was a conventional balloon.
(2) A coating solution was prepared with specific formulations shown in Table 1.
(3) The conventional balloon of the step (1) was immersed in the coating solution of the step (2); the conventional balloon was taken out and placed in an ultraviolet cross-linking apparatus; an ultraviolet cross-linking was performed for 30s, with an intensity of 100%; and after the ultraviolet cross-linking was completed, the conventional balloon was taken out and dried overnight, thereby obtaining a carrier balloon.
(4) Taxol was dispersed in n-hexane and a ultrasonic dispersion was performed for 10 min to prepare a taxol solution; the carrier balloon obtained in the step (3) was immersed in the taxol solution, and the ultrasonic dispersion was performed for another 10 min; and the balloon was taken out and dried for 2 h, thereby obtaining a drug-loaded implantable balloon.
(5) The drug-loaded implantable balloon of the step (4) was immersed in a clear mixture of taxol with ethyl acetate/n-hexane (3:65, v/v) for 5min; the drug-loaded implantable balloon was taken out and dried naturally for 24h, and then was sterilized with ethylene oxide to obtain a crystalline drug coated balloon containing a permanent hydrophilic bottom layer of Example 2; and a crystal morphology was observed using a scanning electron microscope (SEM).

### Example 3

(1) A device body was provided, and the device body of this example was a conventional balloon.
(2) A coating solution was prepared with specific formulations shown in Table 2.
(3) The conventional balloon of the step (1) was immersed in the coating solution of the step (2); the conventional balloon was taken out and placed in an ultraviolet cross-linking apparatus; an ultraviolet cross-linking was performed for 30 s, with an intensity of 100%; and after the ultraviolet cross-linking was completed, the conventional balloon was taken out and dried overnight, thereby obtaining a carrier balloon.
(4) Everolimus was dispersed in n-hexane and a ultrasonic dispersion was performed for 10min to prepare a everolimus solution; the carrier balloon obtained in the step (3) was immersed in the everolimus solution, and the ultrasonic dispersion was performed for another 10min; and the balloon was taken out and dried for 2 h, thereby obtaining a drug-loaded implantable balloon.
(5) The drug-loaded implantable balloon of the step (4) was immersed in a clear mixture of everolimus with ethyl acetate/n-hexane (3:65, v/v) for 5 min; the drug-loaded implantable balloon was taken out and dried naturally for 24 h, and then was sterilized with ethylene oxide to obtain a crystalline drug coated balloon of Example 3, which contains a permanent hydrophilic bottom layer; and a crystal morphology was observed using a scanning electron microscope (SEM).

**Table 2**

| Component | | Material Name | Content % (w/w) |
|---|---|---|---|
| Macromolecula r A-B-C-type block polymer of polymerizable functional groups | A | Polyethylene glycol, molecular weight of 1000 | 24% |
| | B | Chitosan, molecular weight of 5000; graft acrylate, grafting rate of 30% | |
| | C | Dextran, molecular weight of 1000 | |
| Photoinitiator | | 2-Hydroxy-4'-(2-hydroxyethoxy)- 2-methylpheny lacetone | 0.5% |
| photo-cross-linking monomer | | Hydroxyethyl methacrylate | 1% |
| Solvent | | Ethanol | 74.5% |

### Example 4

(1) A device body was provided, and the device body of this example was a metal bracket with a groove on a metal rod.
(2) A coating solution was prepared with specific formulations shown in Table 4.
(3) The metal bracket with the groove of the step (1) was immersed in the coating solution of the step (2); the metal bracket with the groove was taken out and placed in an ultraviolet cross-linking apparatus; an ultraviolet cross-linking was performed for 30 s, with an intensity of 100%; and after the ultraviolet cross-linking was completed, the metal bracket with the groove was taken out and dried overnight, thereby obtaining a carrier bracket.
(4) Everolimus was dispersed in n-hexane and a ultrasonic dispersion was performed for 10 min to prepare a everolimus solution; the carrier bracket obtained in the step (3) was immersed in the everolimus solution, and the ultrasonic dispersion was performed for another 10 min; and the bracket was taken out and dried for 2 h, thereby obtaining a drug-loaded implantable bracket.
(5) The drug-loaded implantable bracket of the step (4) was immersed in a clear mixture of everolimus with ethyl acetate/n-hexane (3:65, v/v) for 5 min; the drug-loaded implantable bracket was taken out and dried naturally for 24 h, and then was sterilized with ethylene oxide to obtain a crystalline drug coated bracket, which contains a permanent hydrophilic bottom layer (drug-loaded implantable carrier); and a crystal morphology was observed using a scanning electron microscope (SEM).

**Table 4**

| Component | | Material Name | Content % (w/w) |
|---|---|---|---|
| Macromolecula r A-B-C-type block polymer of polymerizable functional groups | A | Polyethylene glycol, molecular weight of 1000 | 25% |
| | B | Dextran, molecular weight of 5000; graft acrylate, grafting rate of 30% | |
| | c | Polyvinylpyrrolidone, molecular weight of 5000 | |
| Photoinitiator | | 2-Hydroxy-4'-(2-hydroxyethoxy)- 2-methy lpheny lacetone | 0.5% |
| Solvent | | Ethanol | 74.5% |

### Example 5

(1) A device body was provided, and the device body of this example was a metal bone plate;
(2) A coating solution was prepared with specific formulations shown in Table 3;
(3) The device of the step (1) was immersed in the coating solution of the step (2); the device was taken out and placed in an ultraviolet cross-linking apparatus; an ultraviolet cross-linking was perform for 30 s, with an intensity of 100%; and after the ultraviolet cross-linking was completed, the device was taken out and dry overnight, thereby obtaining a carrier device.
(4) Rapamycin was dispersed in n-hexane and a ultrasonic dispersion was performed for 10 min to prepare a rapamycin solution; the carrier device obtained in the step (3) was immersed in the rapamycin solution, and the ultrasonic dispersion was performed for another 10 min; and the carrier device was taken out and dried for 2 h, thereby obtaining a drug-loaded implantable device.
(5) The drug-loaded implantable device of the step (4) was immersed in a clear mixture of everolimus with ethyl acetate/n-hexane (3:65, v/v) for 5 min; the drug-loaded implantable device was taken out and dried naturally for 24 h, and then was sterilized with ethylene oxide to obtain a crystalline drug coated device containing a permanent hydrophilic bottom layer (drug-loaded implantable carrier); and a crystal morphology was observed using a scanning electron microscope (SEM).

**Table 3**

| Component | | Material Name | Content % (w/w) |
|---|---|---|---|
| Macromolecula r A-B-type block polymer of polymerizable functional groups | A | Polyvinylpyrrolidone, molecular weight of 5000 | 25% |
| | B | Hyaluronic acid, molecular weight of 5000; graft acrylate, grafting rate of 30% | |
| Photoinitiator | | 2-Hydroxy-4'-(2-hydroxyethoxy)- 2-methy lpheny lacetone | 0.5% |
| Solvent | | Ethanol | 74.5% |

### Comparative Example 1

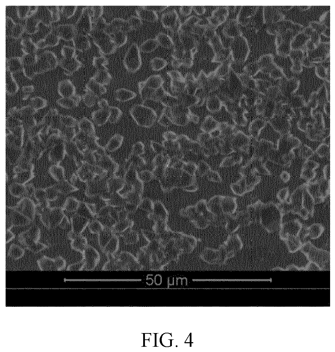

Rapamycin was dispersed in n-hexane to obtain a solution, and then a ultrasonic dispersion was performed for 10 min; a conventional balloon was immersed in the solution, and the ultrasonic dispersion was performed for another 10 min. The balloon was taken out and dried for 2 h. Then, the balloon was immersed in a clear mixture of rapamycin with ethyl acetate/n-hexane (3:65, v/v) for 5 min. The balloon was taken out and dried naturally for 24 h, and then was sterilized with ethylene oxide to obtain a crystalline drug coated balloon. A crystal morphology was observed using a scanning electron microscope (SEM), as shown in FIG. 4.

### Performance test

### (1) Test for firmness of drug-loaded implantable carrier

The drug-loaded implantable balloons of the above Examples 1 to 5 and of the Comparative Example 1 were immersed in phosphate buffer (pH=7.4, 37°C) for 24 h, and then a content of the drug-loaded implantable carriers falling into an immersion solution was measured by a high performance liquid chromatography. The results were shown in Table 3.

**Table 3 Falling Degree of drug-loaded implantable carriers on balloons of different examples**

| | Content of carriers falling into the immersion solution (%, w/w) |
|---|---|
| Example 1 | 0% |
| Example 2 | 0% |
| Example 3 | 0% |
| Example 4 | 0% |
| Example 5 | 0% |

It can be seen that none of carriers in the drug-loaded implantable medical devices of Examples 1 to 5 fall off, which indicates that the drug-loaded implantable carrier of the present disclosure are firmly combined with the device body and are not easy to fall off, so that the toxic and side effects caused by the carrier falling off in human body may be effectively avoided.

### (2) Test for tissue absorption

A segment of isolated porcine arterial blood vessel was kept at a constant temperature of 37°C, and was dilated by a sterilized bare balloon for 1 min, with a pressure of 6 atm, and then the pressure was relieved and the sterilized bare balloon was taken out. The crystalline drug coated devices containing the permanent hydrophilic bottom layer of the Examples 1 to 4 and the crystalline drug coated balloon of the Comparative Example 1 were placed into the dilated blood vessels to respectively dilate the dilated blood vessels for 1 min, with a pressure of 6 atm, and then the pressure was relieved and the balloon and the bracket were taken out. The segments of isolated porcine arterial blood vessels were immediately rinsed with phosphate buffered saline ( PBS) 3 times, with 1mL of PBS each time. Then, a drug concentration in tissues was tested by gas chromatography-mass spectrometer (GC-MS), and a residual drug amount on a surface of the crystalline drug coated device was tested by high performance liquid chromatography (HPLC). The test results were shown in Table 4.

For the metal bone plate containing a permanent hydrophilic bottom layer (drug-loaded implantable carrier) of the Example 5, a porcine carotid artery was kept at a constant temperature of 37°C and was cut along an axial direction; the cut porcine carotid artery was tied and kept on a drug coating surface of the crystalline drug coated bone plate containing a permanent hydrophilic bottom layer (drug-loaded implantable carrier) with a thin thread for 1 min, during which the drug coating was completely covered by the carotid artery; and then the carotid artery was removed by cutting the thin thread, and was rinsed with PBS 3 times, with 1mL of PBS each time. Then, a drug concentration in tissues was tested by gas chromatography-mass spectrometer (GC-MS), and a residual drug amount on a surface of the metal bone plate was tested by high performance liquid chromatography (HPLC). The test results were shown in Table 4.

**Table 4**

| | Drug concentration in tissues (ng/mg) | Residual drug amount on device surface (%) |
|---|---|---|
| Example 1 | 604.8 ± 154.1 ng/mg | 45% |
| Example 2 | 587.5 ± 147.3 ng/mg | 47% |
| Example 3 | 543.7 ± 124.3 ng/mg | 51% |
| Example 4 | 642.5 ± 194.1 ng/mg | 40% |
| Example 5 | 567.1 ± 83.1 ng/mg | 48% |
| Comparative Example 1 | 82.4 ± 50.5 ng/mg | 89% |

As can be seen from Table 4, the drug concentration in tissues of each of the Examples 1 to 5 are much higher than that of the Comparative Example 1, and the residual drug amount on the surface of balloon is much lower than that of the Comparative Example 1.It can be learned that, the drug-loaded implantable medical device of the present disclosure is beneficial to the release of the drug, so that the target drug concentration in the tissues may be reached, and the poor effect caused by the extremely low drug dose can be effectively avoided.

In addition, FIG. 3 shows a crystal morphology of the drug of the crystalline drug coated balloon containing the permanent hydrophilic bottom layer of the Example 1, and FIG. 4 shows a crystal morphology of the drug of the crystalline drug coated balloon of Comparative Example 1. As can be seen from FIG. 3 and FIG. 4, the drug of the Example 1 has an excellent crystalline morphology and a small crystalline size. It can be learned that in the drug-loaded implantable medical device of the present disclosure, the crystalline drug particles with smaller crystal size may be formed, and the risk of embolization may be effectively avoided.

### (3) Test for residence time in tissues

A segment of isolated porcine arterial blood vessel was kept at a constant temperature of 37°C, and was dilated by a sterilized bare balloon for 1 min, with a pressure of 6 atm, and then the pressure was relieved and the sterilized bare balloon was taken out. The crystalline drug coated devices containing the permanent hydrophilic bottom layer of the Examples 1 to 4 and the crystalline drug coated balloon of the Comparative Example 1 were placed into the dilated blood vessels to respectively dilate the dilated blood vessels for 1 min, with a pressure of 6 atm, and then the pressure was relieved and the balloon and the device were taken out. The segments of isolated porcine arterial blood vessels were immediately rinsed with PBS 3 times, with 1mL of PBS each time. Then, the segments of isolated porcine arterial blood vessels were placed and cultured in a culture medium for 7 days and 28 days, respectively, and in triplicate at each time point. After sampling, the drug concentration in tissues were tested by gas chromatography-mass spectrometer (GC-MS). The test results were shown in Table 5.

For the crystalline drug coated device containing a permanent hydrophilic bottom layer (drug-loaded implantable carrier) of the Example 5, a porcine carotid artery was kept at a constant temperature of 37°C and was cut along an axial direction; the cut porcine carotid artery was tied and kept on a drug coating surface of the crystalline drug coated bone plate containing a permanent hydrophilic bottom layer (drug-loaded implantable carrier) with a thin thread for 1 min, during which the drug coating was completely covered by the carotid artery; and then the carotid artery was removed by cutting the thin thread, and was rinsed with PBS 3 times, with 1mL of PBS each time. Then, the porcine carotid artery was placed and cultured in a culture medium for 7 days and 28 days, and in triplicate at each time point. The drug concentrations in tissues were tested by gas chromatography-mass spectrometer (GC-MS). The test results were shown in Table 4.

**Table 5**

| | Drug concentration in tissues (ng/mg) | |
|---|---|---|
| | 7 days | 28 days |
| Example 1 | 508.6 ± 112.4 | 319.3 ± 15.7 |
| Example 2 | 508 ± 148.2 | 302.5 ± 43.4 |
| Example 3 | 438.2 ± 97.1 | 332 ± 31.5 |
| Example 4 | 490.5 ± 122.1 | 387.2 ± 62.9 |
| Example 5 | 470.2 ± 88.5 | 357.2 ± 77.1 |
| Comparative Example 1 | 42.1 ± 23.5 | 7.8 ± 4.9 |

As can be seen from Table 5, the medical devices containing a permanent hydrophilic bottom layer of the Examples 1 to 5 have a significantly better residence time in tissues than Comparative Example 1. It can be learned that the drug-loaded implantable medical device of the present disclosure is beneficial to prolong the residence time in tissues of the drug and has sustained release effect.

Each of the technical features of the above-mentioned embodiments may be combined arbitrarily. To simplify the description, not all the possible combinations of each of the technical features in the above embodiments are described. However, all of the combinations of these technical features should be considered as within the scope of this disclosure, as long as such combinations do not contradict with each other. The above embodiments merely illustrates several embodiments of the present disclosure, and the description thereof is specific and detailed, but it shall not be constructed as limiting the scope of the present disclosure. It should be noted that a plurality of variations and modifications may be made by those skilled in the art without departing from the scope of this disclosure, which are all within the scope of protection of this disclosure. Therefore, the protection scope of this disclosure shall be subject to the appended claims.

## Claims

1. A drug-loaded implantable medical device, comprising a device body, a hydrophilic coating carried on the device body, and crystalline drug particles carried on the hydrophilic coating.

2. The drug-loaded implantable medical device of claim 1, wherein the hydrophilic coating comprises a graft polymer containing photo-cross-linking groups.

3. The drug-loaded implantable medical device of claim 2, wherein the graft polymer containing photo-cross-linking groups is prepared by a graft reaction of a photo-cross-linking monomer and a first polymer;
the photo-cross-linking monomer is at least one of an acrylate-based photo-cross-linking monomer and an acrylamide-based photo-cross-linking monomer; and
the first polymer is a hydrophilic polymer with a side chain containing at least one repeating functional group of hydroxyl group, carboxyl group, and amino group.

4. The drug-loaded implantable medical device of claim 3, wherein the first polymer is selected from at least one of chitosan, dextran, hyaluronic acid, sodium alginate, polyacrylic acid, carbomer, hydroxypropyl methylcellulose, carboxymethylcellulose, polyhydroxyethyl methacrylate, poly (N- (2-hydroxypropyl) methacrylamide), polyarginine, polylysine, and polyaspartate.

5. The drug-loaded implantable medical device of claim 3, wherein the photo-cross-linking monomer is selected from at least one of acrylate, acrylamide and methacrylate.

6. The drug-loaded implantable medical device of claim 1, wherein the hydrophilic coating comprises a block copolymer formed by a graft polymer containing photo-cross-linking groups and a second polymer, and the second polymer is selected from at least one of polyethylene glycol, polyvinyl alcohol, polyvinylpyrrolidone, polyether, polyester, polyamide, polypeptide, and polysaccharide.

7. The drug-loaded implantable medical device of claim 6, wherein a molar ratio of the graft polymer containing photo-cross-linking groups to the second polymer is 1: (0.2~1).

8. The drug-loaded implantable medical device of claim 1, wherein the hydrophilic coating comprises a block copolymer formed by a graft polymer containing photo-cross-linking groups, a second polymer, and a third polymer; the second polymer is selected from at least one of polyethylene glycol, polyvinyl alcohol, polyvinylpyrrolidone, polyether, polyester, polyamide, polypeptide, and polysaccharide; and the third polymer is selected from at least one of polyethylene glycol, polyvinyl alcohol, polyvinylpyrrolidone, polyether, polyester, polyamide, polypeptide, and polysaccharide; and a molar ratio of the graft polymer containing photo-cross-linking groups, the second polymer, and the third polymer is (0.2~1) :1: (0.2~1).

9. The drug-loaded implantable medical device of claim 2, wherein a graft ratio of the graft polymer is in a range from 20% to 60%.

10. The drug-loaded implantable medical device of claim 1, wherein the crystalline drug particles have a particle size in a range from 0.5 µm to 5 µm.

11. The drug-loaded implantable medical device of claim 1, wherein in the crystalline drug particles, a mass percentage of crystalline particles is in a range from 70% to 100%.

12. A method for preparing a drug-loaded implantable medical device, comprising:
providing a device body;
preparing a hydrophilic coating solution;
coating the hydrophilic coating solution on the device body to perform a cross-linking reaction, so as to obtain a carrier medical device, and
carrying a drug onto the carrier medical device to obtain a drug-loaded implantable medical device.

13. The method of claim 12, wherein the hydrophilic coating solution comprises a graft polymer containing photo-cross-linking groups and a photoinitiator.

14. The method of claim 13, wherein the graft polymer containing photo-cross-linking groups is prepared by a graft reaction of a photo-cross-linking monomer and a first polymer;
the photo-cross-linking monomer is at least one of an acrylate-based photo-cross-linking monomer and an acrylamide-based photo-cross-linking monomer; and
the first polymer is a hydrophilic polymer with a side chain containing at least one repeating functional group of hydroxyl group, carboxyl group, and amino group.

15. The method of claim 14, wherein the first polymer is selected from at least one of chitosan, dextran, hyaluronic acid, sodium alginate, polyacrylic acid, carbomer, hydroxypropyl methylcellulose, carboxymethylcellulose, polyhydroxyethyl methacrylate, poly (N- (2-hydroxypropyl) methacrylamide), polyarginine, polylysine, and polyaspartate; and
the photo-cross-linking monomer is selected from at least one of acrylate, acrylamide and methacrylate.

16. The method of claim 13, wherein the hydrophilic coating solution further comprises a second polymer, and the second polymer is selected from at least one of polyethylene glycol, polyvinyl alcohol, polyvinylpyrrolidone, polyether, polyester, polyamide, polypeptide, and polysaccharide.

17. The method of claim 16, wherein a molar ratio of the graft polymer containing photo-cross-linking groups to the second polymer is 1: (0.2~1).

18. The method of claim 12, wherein the hydrophilic coating solution further comprises a photo-cross-linking monomer, and the photo-cross-linking monomer is selected from at least one of acrylate, acrylamide and methacrylate.

19. The method of claim 12, wherein the drug is carried onto the carrier medical device by an immersion method.

20. The method of claim 19, wherein the carrying a drug onto the carrier medical device by an immersion method comprises:
preparing a drug solution;
immersing the carrier medical device in the drug solution; and
taking out the immersed carrier medical device, and then immersing the immersed carrier medical device in a crystallization solution for crystallization, and then drying the carrier medical device.
